# EUROPEAN PATENT SPECIFICATION

(11) **EP 3 299 007 B1**
(45) Date of publication and mention of the grant of the patent: **14.10.2020**
(21) Application number: 15849822.0
(22) Date of filing: 11.11.2015
(51) Int. Cl.: A61H 39/02

(54) **APPARATUS, SYSTEM AND METHOD FOR REMOTELY DETERMINING ACUPUNCTURE POINT**
VORRICHTUNG, SYSTEM UND VERFAHREN ZUR FERNBESTIMMUNG VON AKUPUNKTURPUNKTEN
APPAREIL,SYSTÈME ET PROCÉDÉ DE DÉTERMINATION À DISTANCE DE POINT D'ACUPUNCTURE

(30) Priority: 22.05.2015 CN 201510268800
(43) Date of publication of application: 28.03.2018
(73) Proprietor: BOE Technology Group Co., Ltd., Beijing 100015 (CN)
(72) Inventor: CHAI, Dong, Beijing 100176 (CN); WANG, Hong, Beijing 100176 (CN); SONG, Jian, Beijing 100176 (CN); LI, Linzhe, Beijing 100176 (CN); LV, Xuewen, Beijing 100176 (CN)
(74) Representative: Jacobacci Coralis Harle
(86) International application number: PCT/CN2015/094247
(87) International publication number: WO 2016/188046

(56) References cited:
- EP-A1- 1 426 026
- CN-A- 102 698 371
- CN-A- 104 523 420
- CN-A- 104 800 068
- JP-A- 2002 078 772
- KR-A- 20090 020 051
- US-A- 5 417 212
- US-A1- 2013 027 368

## Description

### TECHNICAL FIELD

The disclosure relates to an apparatus, a system and a method for remotely determining acupuncture point.

### BACKGROUND OF THE INVENTION

The determination of acupuncture point has a significant impact on the treatment of diseases when curing some diseases. A professional doctor is required to determine the acupuncture point on a target person's body, and then applies acupuncture at the acupuncture point to cure some diseases. A person without professional training cannot determine the position of the acupuncture point to be acupunctured on the target person's body, therefore the target person needs to go to hospital when the acupuncture point of the target person is required to be determined, which may be very inconvenient for the target person unable to move freely.

Closest prior art US2013027368A discloses an apparatus and method for determining and displaying acupuncture points on an uneven external body surface via three-dimensionally scanning. The acupuncture point determination apparatus and method of KR20090020051A has a projection agent unit and projection unit for reflecting multiple images to partial body of a patient. Finally, JP2002078772A discloses a remote computer diagnostic image and laser acupuncture therapeutic apparatus and method.

Therefore, how to facilitate the target person unable to move freely to determine the acupuncture point on his body has become a technical problem to be solved in the field.

### SUMMARY OF THE INVENTION

The invention aims at providing an apparatus and method for remotely determining acupuncture point and a system including the apparatus. Ordinary nursing personnel can accurately determine the position of the acupuncture point by utilizing the system, therefore the target person does not have to go to hospital, and the acupuncture point on the body of the target person can be determined by the ordinary nursing personnel at home.

There is provided an apparatus for remotely determining acupuncture point according to claim 1.

The apparatus comprises a first controller, an image acquisition module, a first image transport module and a positioning mark generation module, wherein the image acquisition module is used for acquiring a target image of a part of the body of a target person on which the acupuncture point is required to be determined; the first image transport module is used for transmitting the target image to a remote device; the first image transport module is further used for transmitting the received target image marked with a first mark to the first controller; the first controller determining a position coordinate of the acupuncture point according to the target image marked with the first mark and controlling the positioning mark generation module to generate a second mark at the position coordinate of the acupuncture point according to the position coordinate.

According to the invention, the position coordinate of the acupuncture point is a three-dimensional coordinate, and the first controller comprises a control unit and a three-dimensional coordinate conversion unit, wherein the control unit can control the three-dimensional coordinate conversion unit to convert a two-dimensional coordinate of the first mark in the target image marked with the first mark to the three-dimensional coordinate within a space in which the target person is located.

According to the invention, the image acquisition module can further acquire in real time a reference image of the part of the body of the target person on which the acupuncture point is required to be determined, the first controller further comprises a deciding unit, wherein the deciding unit is used for deciding whether the reference image is consistent with the target image and sending a signal indicating consistency to the controller unit when the reference image and the target image are consistent, the controller unit, after receiving the signal indicating consistency, sends a signal indicating start of conversion to the three-dimensional coordinate conversion unit, and the three-dimensional coordinate conversion unit converts the two-dimensional coordinate of the first mark to the three-dimensional coordinate after receiving the signal indicating start of conversion.

According to an embodiment, the first controller further comprises a relationship transformation unit, wherein the deciding unit sends a signal indicating inconsistency to the controller unit when the reference image is inconsistent with the target image, the controller unit, after receiving the signal indicating inconsistency, can send a signal indicating start of transformation to the relationship transformation unit, the relationship transformation unit determines a relationship between a two-dimensional coordinate system in the target image and a three-dimensional space in which the target person is located according to the signal indicating start of transformation, and the three-dimensional coordinate conversion unit obtains the three-dimensional coordinate of the second mark according to the relationship.

According to an embodiment, the positioning mark generation module comprises a drive motor and a light pointer, wherein the light pointer can emit an optical signal, a controlling end of the drive motor is connected with the first controller, the drive motor, after receiving the position coordinate of the acupuncture point, can drive the light pointer to move to cause the light pointer to generate the second mark at the position of the acupuncture point.

According to an embodiment, the light pointer comprises projector or laser pointer.

There is provided a system for remotely determining acupuncture point according to claim 5. The system comprises the above mentioned apparatus for remotely determining acupuncture point provided by the disclosure and a remote device, wherein the remote device comprises a second image transport module for communicating with the first image transport module and a mark writing module for adding the first mark on the received target image.

According to an embodiment, the first image transport module can send the target image acquired by the image acquisition module to the second image transport module which is used for receiving the target image and can send the target image with the first mark to the first image transport module.

The nursing personnel unable to determine the position of the acupuncture point can also accurately determine the acupuncture point of the target person by utilizing the apparatus and system provided by the disclosure, therefore the target person does not have to leave their lodgings and go to hospital

Further according to the invention, there is provided a method for remotely determining acupuncture points according to claim 7.

### BRIEF DESCRIPTION OF THE DRAWINGS

The accompanying drawings is provided for further understanding the present invention, constitutes a part of the specification and is used for explaining the present invention in connection with embodiments, but is not aimed to limit the disclosure, in which:
Figure 1 schematically shows a block diagram of an apparatus for remotely determining acupuncture point in accordance with an embodiment of the disclosure; and
Figure 2 shows a schematic diagram of a system for remotely determining acupuncture point in accordance with an embodiment of the disclosure.

### DETAILED DESCRIPTION OF SPECIFIC EMBODIMENTS

To make those skilled in the art to better understand the objects, technical solutions and advantages of embodiments of the disclosure, the technical solution of embodiments of the disclosure will be described clearly and completely in connection with the accompanying drawings of embodiments of the disclosure. It is obvious that the described embodiments are only some, but not all the embodiments of the invention defined by the appended claims.

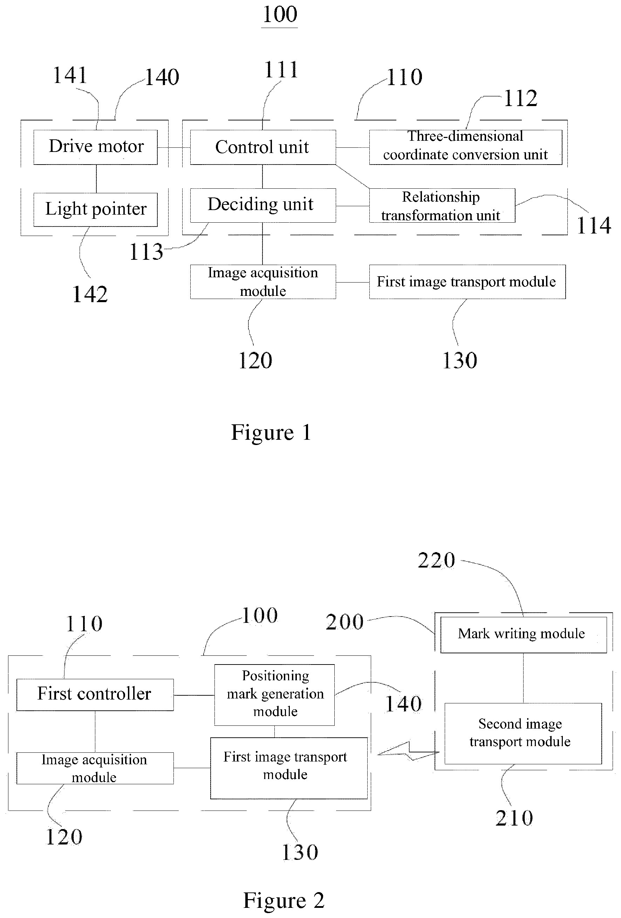

As shown in Fig.1 and Fig.2, as an embodiment of the disclosure, there is provided an apparatus for remotely determining acupuncture point. The apparatus includes a first controller 110, an image acquisition module 120, a first image transport module 130 and a positioning mark generation module 140.

The image acquisition module 120 is used for acquiring a target image of a part of the body of a target person on which the acupuncture point is required to be determined. The image acquisition module 120 may acquire a two-dimensional target image and/or a three-dimensional target image. The first image transport module 130 is used for transmitting the target image acquired by the image acquisition module 120 to a remote device 200.

In the invention, the apparatus for remotely determining acupuncture point is arranged at a side of the target person, and the apparatus 100 is operated by the nursing personnel at the side of the target person or by the target person. The disclosure does not make the specific stipulation to the concrete structure of the image acquisition module 120, for example, the image acquisition module 120 may be an image acquisition apparatus such as a high definition camera or the like. The apparatus 100 may be operated by the nursing personnel at the side of the target person, for example, the nursing personnel, after starting the apparatus 100, can utilize the image acquisition module 120 to take a picture of a part of the body of the target person to be acupunctured to obtain the target image. It can be easily understood that the nursing personnel, before utilizing the image acquisition module 120 to acquire the target image, needs to communicate with a doctor and informs the doctor of the specific condition of the target person, and the doctor decides which acupuncture points of the target person are required to be treated and then informs the nursing personnel of the approximate positions of the acupuncture points on a human body. The first image transport module 130 is used for transmitting the target image to the remote device 200 after the image acquisition module 120 acquires the target image. The doctor at a side of the remote device 200 analyses the target image received by the remote device 200 to determine the exact position of the acupuncture point to be acupunctured, and then marks the exact position (i.e., the first mark) of the acupuncture point on the target image. The exact position may be corresponding to a two-dimensional coordinate or a three-dimensional coordinate depending on whether the target image is a two-dimensional target image or a three-dimensional target image. The image marked with the position (i.e., the first mark) of the acupuncture point is transmitted to the apparatus 100 at the side of the target person.

The first image transport module 130 in the apparatus 100 is further used for transmitting the received target image marked with the first mark to the first controller 110 which determines a position coordinate of the acupuncture point according to the target image marked with the first mark and controls the positioning mark generation module 140 to generate a second mark at the position coordinate of the acupuncture point according to the position coordinate.

It is noted that the second mark herein is located on the body of the target person and the nursing personnel applies acupuncture at the second mark.

The nursing personnel unable to determine the position of the acupuncture point can also accurately determine the acupuncture point of the target person through the above description, therefore the target person does not have to leave their lodgings and go to hospital.

In the invention, the position coordinate of the acupuncture point determined by the first controller 110 is a three-dimensional coordinate, and the positioning mark generation module 140 may form a positioning mark at the three-dimensional coordinate representing the position of the acupuncture point. In an embodiment, the target image and the target image marked with the first mark are transmitted in a way of two-dimensional image, in other words, the doctor at the side of the remote device would mark the first mark on a two-dimensional target image to form a new two-dimensional image, and the remote device 200 would transmit the new two-dimensional image to the apparatus 100. In order to form the second mark within a three-dimensional space in which the target person is located, the first controller 110 may include a control unit 111 and a three-dimensional coordinate conversion unit 112 as shown in Fig.1. The control unit 111 can control the three-dimensional coordinate conversion unit 112 to convert a two-dimensional coordinate of the first mark in the target image marked with the first mark to the three-dimensional coordinate within a space in which the target person is located. The control unit 111 further controls the positioning mark generation module 140 to form the second mark at the above mentioned three-dimensional coordinate.

In general, a person cannot maintain a same action for a long time. In other words, the target person may not maintain the posture in the target image any more when the target image marked with the first mark is transmitted to the side of the target person.

In the invention, a real time posture of the target person can also be decided after the target image marked with the first mark is received. When the real time posture of the target person is consistent with the posture in the target image after the target image marked with the first mark is received, a two-dimensional coordinate of the first mark in the target image can be directly converted to a three-dimensional coordinate in a three-dimensional space in which the target person is located if the first mark is represented by the two-dimensional coordinate; a three-dimensional coordinate can be directly used if the first mark is represented by the three-dimensional coordinate. Correspondingly, the image acquisition module 120 can further acquire in real time a reference image of the part of the body of the target person on which the acupuncture point is required to be determined, wherein the reference image may be a two- or three-dimensional reference image. As shown in Fig.1, the first controller 110 further includes a deciding unit 113, wherein the deciding unit 113 is used for deciding whether the reference image is consistent with the target image and sending a signal indicating consistency to the controller unit 111 when the reference image and the target image are consistent. The controller unit 111, after receiving the signal indicating consistency, may send a signal indicating start of conversion to the three-dimensional coordinate conversion unit 112 if the first mark is represented by a two-dimensional coordinate, which would convert the two-dimensional coordinate of the first mark to the three-dimensional coordinate after receiving the signal indicating start of conversion. If the first mark is represented by a three-dimensional coordinate, the three-dimensional coordinate can be directly used.

When the posture of the target person shown in the reference image is not consistent with the posture in the target image, the nursing personnel may adjust the posture of the target person such that the target person may return to the posture shown in the target image.

For the convenience of the target person, the target image marked with the first mark instead of the posture of the target person may be adjusted. In an embodiment, the first controller 110 may further includes a relationship transformation unit 114, wherein the deciding unit 113 sends a signal indicating inconsistency to the control unit 111 which can send a signal indicating start of transformation to the relationship transformation unit 114 after receiving the signal indicating inconsistency, the relationship transformation unit 114, after receiving the signal indicating start of transformation, determines a relationship between a two-dimensional coordinate system in the target image and a three-dimensional space in which the target person is located if the target image is a two-dimensional target image, and the three-dimensional coordinate conversion unit obtains the three-dimensional coordinate of the second mark according to the relationship. If the target image is a three-dimensional target image, the three-dimensional coordinate conversion unit may determine the three-dimensional coordinate of the second mark according to the location movement between the target person in the target image and the target person in the reference image.

In an embodiment, the target image is a two-dimensional target image, and the relationship transformation unit 114 may analyze the muscle and body outline of the target person to obtain a transformation relationship between the target image marked with the first mark and the reference image when the target image marked with the first mark is adjusted, after this, the three-dimensional coordinate conversion unit converts the two-dimensional coordinate of the first mark to the three-dimensional coordinate of the second mark according to the above transformation relationship.

It can be easily understood that the three-dimensional coordinate conversion unit 112 uses the two-dimensional coordinate or the three-dimensional coordinate of the first mark in the adjusted target image to generate the three-dimensional coordinate of the second mark. It can be further convenient to the target person without the adjustment of posture of the target person by the arrangement of the relationship transformation unit 114.

There is not a particular requirement for the specific structure of the positioning mark generation module in the disclosure only if it can form the second mark at the above three-dimensional coordinate. The second mark may be an optical signal mark or other entity mark. When the second mark is an entity mark such as a small ball, the positioning mark generation module may further include a drive apparatus for driving the entity mark to the three-dimensional coordinate.

In an embodiment, for accurate positioning, the positioning mark generation module 140 may include a drive motor 141 and a light pointer 142, wherein the light pointer 142 can emit an optical signal, a controlling end of the drive motor 141 is connected with the first controller 110, the drive motor 141 can drive the light pointer 142 to move after receiving the position coordinate of the acupuncture point, to cause the light pointer 142 to generate the second mark at the position of the acupuncture point.

In this embodiment, the second mark may be a light spot projecting on the body of the target person. The smaller an area of the light spot is, the more accurate the position of the mark is. In an embodiment, in order to obtain a smaller light spot, the light pointer 141 may include projector or laser pointer.

Another embodiment of the invention further provides a system for remotely determining acupuncture point. As shown in Fig.2, the system includes the above mentioned apparatus 100 for remotely determining acupuncture point provided by the disclosure and a remote device 200, wherein the remote device 200 includes a second image transport module 210 for communicating with the first image transport module 120 and a mark writing module 220 for adding the first mark on the received target image.

The remote device 200 is operated by a professional doctor as described above, and may be a personal computer comprising a second controller for controlling the running and operation of the image transport module 210 and the mark writing module 220. The mark writing module 220 may be an input device such as keyboard, mouse, digital panel, or the like.

As mentioned above, in the system provided by the disclosure, the apparatus 100 may convert the position coordinate of the acupuncture point to the second mark in a three-dimensional space in which the target person is located after the doctor at the side of the remote device marks the position of the acupuncture point on the target image, wherein the second mark is on the body of the target person and the nursing personnel can apply acupuncture at the second mark. Therefore the system provided by the disclosure is especially suitable for determining the acupuncture point of the body of the target person unable to move freely.

In the disclosure, the apparatus 100 communicates with the remote device 200 through Internet.

A flowchart of determining the acupuncture point of the target person by utilizing the system provided by the disclosure is described in the following. In this embodiment, the image acquisition module is a high definition camera or any other suitable image acquisition device.

Utilizing the system provided by the disclosure to determine the acupuncture point may include the following steps:
acquiring a target image by utilizing the high definition camera;
transmitting the target image to a remote device, wherein the target image is viewed by a professional doctor at the side of the remote device;
when the professional doctor is able to determine the position of the acupuncture point through the target image, the professional doctor selects the position of the acupuncture point on the target image displayed by the remote device through a mouse pointer to form a first mark; when the professional doctor is unable to determine the position of the acupuncture point through the target image, the apparatus at the side of the patient is required to reacquire an image and send it to the remote device until the professional doctor can determine the position of the acupuncture point;
the doctor returns the target image marked with the first mark to the apparatus for remotely determining acupuncture point;
the target image marked with the first mark is compared with the a reference image to decide whether the postures of patient in the two images are same;
when the postures of patient in the two images are same, the two-dimensional coordinate of the first mark in the target image can be converted to the three-dimensional coordinate within a three-dimensional space in which the target person is located through the three-dimensional coordinate conversion unit; when the postures of patient in the two images are not same, the muscle and body outline of the target person is analyzed to obtain a relationship between the target image and the reference image through the relationship transformation unit, then the three-dimensional coordinate conversion unit determines the three-dimensional coordinate of the second mark according to the relationship;
the control unit controls the operation of the drive motor, thereby driving the light pointer to emit a light to form the second mark on the surface of the body of the target person, wherein the coordinate of the second mark is the three-dimensional coordinate as mentioned above;
finally, the nursing personnel applies acupuncture at the second mark.

It may be understood that the above mentioned embodiments are only exemplary embodiments for the illustration of the principle of the invention. Various modifications and variations can be made by those skilled in the art without departing from the scope of the invention defined by the appended claims.

## Claims

1. An apparatus (100) for remotely determining acupuncture point, wherein the apparatus comprises a first controller (110), an image acquisition module (120), a first image transport module (130) and a positioning mark generation module (140),
the image acquisition module (120) used for taking a target image of a part of the body of a target person on which the acupuncture point is required to be determined;
the first image transport module (130) used for transmitting the target image to a remote device and receiving the target image marked with a first mark from the remote device;
the first image transport module (130) further used for transmitting the received target image marked with a first mark to the first controller (110);
the first controller (110) is configured to determine a position coordinate of the acupuncture point according to the target image marked with the first mark, and is configured to control the positioning mark generation module (140) to generate a second mark at the position coordinate of the acupuncture point according to the position coordinate, wherein the position coordinate of the acupuncture point is a three-dimensional coordinate, and the first controller (110) comprises a control unit (111) and a three-dimensional coordinate conversion unit (112), wherein the control unit (111) is configured to control the three-dimensional coordinate conversion unit to convert a two-dimensional coordinate of the first mark in the target image marked with the first mark to the three-dimensional coordinate within a space in which the target person is located, wherein the image acquisition module (120) is configured to further take in real time a reference image of the part of the body of the target person on which the acupuncture point is required to be determined, the first controller (110) further comprises a deciding unit (113), wherein the deciding unit (113) is used for deciding whether the reference image is consistent with the target image and sending a signal indicating consistency to the controller unit when the reference image and the target image are consistent, the controller unit (111), after receiving the signal indicating consistency, is configured to send a signal indicating start of conversion to the three-dimensional coordinate conversion unit, and the three-dimensional coordinate conversion unit (112) is configured to convert the two-dimensional coordinate of the first mark to the three-dimensional coordinate after receiving the signal indicating start of conversion.

2. The apparatus according to claim 1, wherein the first controller (110) further comprises a relationship transformation unit (114), the deciding unit (113) is configured to send a signal, indicating inconsistency to the controller unit (111) when the reference image is inconsistent with the target image, the controller unit (111), after receiving the signal indicating inconsistency, is configured to send send a signal indicating start of transformation to the relationship transformation unit (114), the relationship transformation unit (114) is configured to determine a relationship between a two-dimensional coordinate system in the target image and a three-dimensional space in which the target person is located according to the signal indicating start of transformation, and the three-dimensional coordinate conversion unit (112) is configured to obtain the three-dimensional coordinate of the second mark according to the relationship.

3. The apparatus according to any one of claims 1-2, wherein the positioning mark generation module (140) comprises a drive motor (141) and a light pointer (142), the light pointer (142) is configured to emit an optical signal, a controlling end of the drive motor (141) is connected with the first controller (110), the drive motor (141), after receiving the position coordinate of the acupuncture point, is configured to drive the light pointer (142) to move to cause the light pointer (142) to generate the second mark at the position of the acupuncture point.

4. The apparatus according to claim 3, wherein the light pointer (142) comprises projector or laser pointer.

5. A system for remotely determining acupuncture point, comprising the apparatus (110) for remotely determining acupuncture point according to any one of claims 1-4 and a remote device (200), wherein the remote device (200) comprises a second image transport module (210) for communicating with the first image transport module and a mark writing module (220) for adding the first mark on the received target image.

6. The system according to claim 5, wherein the first image transport module (130) is configured to send the target image took by the image acquisition module (120) to the second image transport module (210) which is used for receiving the target image and can send the target image with the first mark to the first image transport module (130).

7. A method for remotely determining acupuncture point, comprising:
acquiring a target image of a part of the body of a target person on which the acupuncture point is required to be determined;
transmitting the target image to a remote device;
receiving the target image marked with a first mark from the remote device;
determining a position coordinate of the acupuncture point according to the target image marked with the first mark; and
generating a second mark at the position coordinate of the acupuncture point according to the position coordinate, wherein the position coordinate of the acupuncture point is a three-dimensional coordinate, and the method further comprises:
acquiring in real time a reference image of the part of the body of the target person on which the acupuncture point is required to be determined;
deciding whether the reference image is consistent with the target image; and
converting the two-dimensional coordinate of the first mark to the three-dimensional coordinate when the reference image and the target image are consistent.

8. The method according to claim 7, further comprising:
determining a relationship between a two-dimensional coordinate system in the target image and a three-dimensional space in which the target person is located when the reference image is inconsistent with the target image; and
obtaining the three-dimensional coordinate of the second mark according to the relationship.

9. The method according to any one of claims 7-8, further comprising:
driving a light pointer to move to cause the light pointer to generate the second mark at the position of the acupuncture point.

10. The method according to claim 9, wherein the light pointer comprises projector or laser pointer.

## Patentansprüche

1. Gerät (100) zur Fernbestimmung eines Akupunkturpunkts, wobei
das Gerät eine erste Steuerung (110), ein Bilderfassungsmodul (120), ein erstes Bildtransportmodul (130) und ein Modul (140) zur Erzeugung einer Positionsmarkierung aufweist,
das Bilderfassungsmodul (120) dazu benutzt wird, ein Zielbild von einem Teil des Körpers einer Zielperson, auf dem der Akupunkturpunkt bestimmt werden soll, zu machen,
das erste Bildtransportmodul (130) dazu benutzt wird, das Zielbild an eine entfernte Vorrichtung zu übermitteln und das mit einer ersten Markierung versehene Zielbild von der entfernten Vorrichtung zu empfangen,
das erste Bildtransportmodul (130) außerdem dazu benutzt wird, das empfangene, mit der ersten Markierung versehene Zielbild an die erste Steuerung (110) zu übermitteln,
die erste Steuerung (110) dazu ausgelegt ist, eine Positionskoordinate des Akupunkturpunkts gemäß dem mit der ersten Markierung versehenen Zielbild zu bestimmen, und dazu ausgelegt ist, das Modul (140) zur Erzeugung einer Positionsmarkierung zu steuern, um eine zweite Markierung an der Positionskoordinate des Akupunkturpunkts gemäß der Positionskoordinate zu erzeugen, wobei die Positionskoordinate des Akupunkturpunkts eine dreidimensionale Koordinate ist und die erste Steuerung (110) eine Steuereinheit (111) und eine Umwandlungseinheit (112) für dreidimensionale Koordinaten aufweist, wobei die Steuereinheit (111) dazu ausgelegt ist, die Umwandlungseinheit für dreidimensionale Koordinaten zu steuern, um eine zweidimensionale Koordinate der ersten Markierung auf dem mit der ersten Markierung versehenen Zielbild in die dreidimensionale Koordinate in einem Raum, in dem sich die Zielperson befindet, umzuwandeln, wobei das Bilderfassungsmodul (120) dazu ausgelegt ist, außerdem in Echtzeit ein Referenzbild von Teil des Körpers der Zielperson, auf dem der Akupunkturpunkt bestimmt werden soll, zu machen, wobei die erste Steuerung (110) außerdem eine Entscheidungseinheit (113) aufweist, wobei die Entscheidungseinheit (113) dazu benutzt wird, zu entscheiden, ob das Referenzbild zu dem Zielbild paßt, und ein Signal auszusenden, das der Steuereinheit das Zusammenpassen anzeigt, wenn das Referenzbild zu dem Zielbild paßt, wobei die Steuereinheit (111) dazu ausgelegt ist, nach Empfang des das Zusammenpassen anzeigenden Signals ein Signal zum Starten der Umwandlung an die Umwandlungseinheit (112) für dreidimensionale Koordinaten zu senden, und wobei die Umwandlungseinheit (112) für dreidimensionale Koordinaten dazu ausgelegt ist, nach Erhalt des Signals, das das Starten der Umwandlung anzeigt, die zweidimensionalen Koordinate der ersten Markierung in eine dreidimensionale Koordinate umzuwandeln.

2. Gerät gemäß Anspruch 1, **dadurch gekennzeichnet, daß** die erste Steuerung (110) außerdem eine Einheit (114) zur Umwandlung des Bezugs aufweist, wobei die Entscheidungseinheit (113) dazu ausgelegt ist, an die Steuereinheit (111) ein ein fehlendes Passen anzeigendes Signal zu senden, wenn das Referenzbild nicht zu dem Zielbild paßt, wobei die Steuereinheit (111) dazu ausgelegt ist, nach Erhalt des das fehlende Passen anzeigenden Signals an die Einheit (114) zur Umwandlung des Bezugs ein Signal zum Starten einer Umwandlung zu senden, wobei die Einheit (114) zur Umwandlung des Bezugs dazu ausgelegt ist, gemäß dem Signal, das das Starten der Umwandlung anzeigt, einen Bezug zwischen einem zweidimensionalen Koordinatensystem im Zielbild und einem dreidimensionalen Raum, in dem sich die Zielperson befindet, zu bestimmen, und wobei die Umwandlungseinheit (112) für dreidimensionale Koordinaten dazu ausgelegt ist, die dreidimensionalen Koordinate der zweiten Markierung gemäß des Bezugs zu erhalten.

3. Gerät gemäß einem der Ansprüche 1 bis 2, **dadurch gekennzeichnet, daß** das Modul (140) zur Erzeugung einer Positionsmarkierung einen Antriebsmotor (141) und einen Lichtzeiger (142) aufweist, wobei der Lichtzeiger (142) dazu ausgelegt ist, ein optisches Signal auszusenden, wobei ein Steuerungsende des Antriebsmotors (141) mit der ersten Steuerung (110) verbunden ist, wobei der Antriebsmotor (141) dazu ausgelegt ist, nach Erhalt der Positionskoordinate des Akupunkturpunkts den Lichtzeiger (142) anzutreiben, um den Lichtzeiger (142) dazu zu bringen, die zweite Markierung an der Position des Akupunkturpunkts zu erzeugen.

4. Gerät gemäß Anspruch 3, **dadurch gekennzeichnet, daß** der Lichtzeiger (142) einen Beamer oder einen Laserpointer aufweist.

5. System zur Fernbestimmung eines Akupunkturpunkts, das das Gerät zur Fernbestimmung eines Akupunkturpunkts gemäß einem der Ansprüche 1 bis 4 und eine entfernte Vorrichtung (200) aufweist, wobei die entfernte Vorrichtung (200) ein zweites Bildtransportmodul (210) zum Kommunizieren mit dem ersten Bildtransportmodul und ein Modul (220) zum Schreiben von Markierungen, um auf dem empfangenen Zielbild die erste Markierung hinzuzufügen, aufweist

6. System gemäß Anspruch 5, **dadurch gekennzeichnet, daß** das erste Bildtransportmodul (130) dazu ausgelegt ist, das vom Bilderfassungsmodul (120) erfaßte Zielbild an das zweite Bildtransportmodul (210), das dazu benutzt wird, das Zielbild zu empfangen, und das das Zielbild mit der ersten Markierung an das erste Bildtransportmodul (130) senden kann, zu senden.

7. Verfahren zur Fernbestimmung eines Akupunkturpunkts mit Erfassen eines Zielbilds eines Teils des Körpers einer Zielperson, auf dem der Akupunkturpunkt erfaßt werden soll,
Übermitteln des Zielbilds an eine entfernte Vorrichtung,
Empfangen des mit einer ersten Markierung versehenen Zielbilds von der entfernten Vorrichtung,
Bestimmen einer Positionskoordinate des Akupunkturpunkts gemäß dem mit der ersten Markierung markierten Zielbild, und
Erzeugen einer zweiten Markierung an der Positionskoordinate des Akupunkturpunkts gemäß der Positionskoordinate, wobei die Positionskoordinate des Akupunkturpunkts eine dreidimensionale Koordinate ist und das Verfahren außerdem
Erfassen in Echtzeit eines Referenzbilds des Teils des Körpers der Zielperson, auf dem der Akupunkturpunkt bestimmt werden soll,
Entscheiden, ob das Referenzbild zu dem Zielbild paßt, und
Umwandeln der zweidimensionalen Koordinate der ersten Markierung in die dreidimensionale Koordinate, wenn das Referenzbild zu dem Zielbild paßt, aufweist.

8. Verfahren gemäß Anspruch 7, das außerdem
Bestimmen eines Bezugs zwischen einem zweidimensionalen Koordinatensystem im Zielbild und einem dreidimensionalen Raum, in dem sich die Zielperson befindet, wenn das Referenzbild nicht zu dem Zielbild paßt, und
Erhalten der dreidimensionalen Koordinate der zweiten Markierung gemäß dem Bezug
aufweist.

9. Verfahren gemäß einem der Ansprüche 7 bis 8, das außerdem
Antreiben eines Lichtzeigers, um den Lichtzeiger dazu zu bringen, die zweite Markierung an der Position des Akupunkturpunkts zu erzeugen,
aufweist.

10. Verfahren gemäß Anspruch 9, **dadurch gekennzeichnet, daß** der Lichtzeiger einen Beamer oder einen Laserpointer aufweist.

## Revendications

1. Appareil (100) de détermination à distance d'un point d'acuponcture, l'appareil comprenant :
un premier contrôleur (110), un module d'acquisition d'image (120), un premier module de transport d'image (130) et un module de génération de marque de positionnement (140),
le module d'acquisition d'image (120) étant utilisé pour prendre une image cible d'une partie du corps d'une personne cible sur laquelle le point d'acuponcture doit être déterminé ;
le premier module de transport d'image (130) étant utilisé pour transmettre l'image cible à un dispositif distant et recevoir l'image cible marquée d'une première marque en provenance du dispositif distant ;
le premier module de transport d'image (130) étant en outre utilisé pour transmettre au premier contrôleur (110) l'image cible reçue marquée d'une première marque ;
le premier contrôleur (110) étant configuré pour déterminer des coordonnées de position du point d'acuponcture d'après l'image cible marquée de la première marque, et étant configurée pour commander le module de génération de marque de positionnement (140) pour générer une deuxième marque aux coordonnées de position du point d'acuponcture d'après les coordonnées de position, les coordonnées de position du point d'acuponcture étant des coordonnées tridimensionnelles, et le premier contrôleur (110) comprenant une unité de commande (111) et une unité de conversion en coordonnées tridimensionnelles (112), l'unité de commande (111) étant configurée pour commander l'unité de conversion en coordonnées tridimensionnelles pour convertir des coordonnées bidimensionnelles de la première marque dans l'image cible marquée de la première marque en les coordonnées tridimensionnelles dans un espace dans lequel se trouve la personne cible, le module d'acquisition d'image (120) étant configuré pour prendre en outre en temps réel une image de référence de la partie du corps de la personne cible sur laquelle le point d'acuponcture doit être déterminé, le premier contrôleur (110) comprenant en outre une unité de décision (113), l'unité de décision (113) étant utilisée pour décider sur fait que l'image de référence est ou non cohérente avec l'image cible et envoyer un signal d'indication de cohérence à l'unité de commande lorsque l'image de référence et l'image cible sont cohérentes, l'unité de commande (111) étant configurée pour, après avoir reçu le signal d'indication de cohérence, envoyer un signal d'indication de démarrage de conversion à l'unité de conversion en coordonnées tridimensionnelles, et l'unité de conversion en coordonnées tridimensionnelles (112) étant configurée pour convertir les coordonnées bidimensionnelles de la première marque en les coordonnées tridimensionnelles après avoir reçu le signal d'indication de démarrage de conversion.

2. Appareil selon la revendication 1, dans lequel le premier contrôleur (110) comprend en outre une unité de transformation de relation (114), l'unité de décision (113) étant configurée pour envoyer un signal d'indication de non-cohérence à l'unité de commande (111) lorsque l'image de référence n'est pas cohérente avec l'image cible, l'unité de commande (111) étant configurée pour, après avoir reçu le signal d'indication de non-cohérence, envoyer un signal d'indication de démarrage de transformation à l'unité de transformation de relation (114), l'unité de transformation de relation (114) étant configurée pour déterminer une relation entre un système de coordonnées bidimensionnelles dans l'image cible et un espace tridimensionnel dans lequel se trouve la personne cible d'après le signal d'indication de démarrage de transformation, et l'unité de conversion en coordonnées tridimensionnelles (112) étant configurée pour obtenir les coordonnées tridimensionnelles de la deuxième marque d'après la relation.

3. Appareil selon l'une quelconque des revendications 1 à 2, dans lequel le module de génération de marque de positionnement (140) comprend un moteur d'entraînement (141) et un pointeur lumineux (142), le pointeur lumineux (142) étant configuré pour émettre un signal optique, une extrémité de commande du moteur d'entraînement (141) étant connectée au premier contrôleur (110), le moteur d'entraînement (141) étant configuré pour, après avoir reçu les coordonnées de position du point d'acuponcture, entraîner le pointeur lumineux (142) à se déplacer pour faire en sorte que le pointeur lumineux (142) génère la deuxième marque à la position du point d'acuponcture.

4. Appareil selon la revendication 3, dans lequel le pointeur lumineux (142) comprend un projecteur ou un pointeur laser.

5. Système de détermination à distance d'un point d'acuponcture, comprenant l'appareil (110) de détermination à distance d'un point d'acuponcture selon l'une quelconque des revendications 1 à 4 et un dispositif distant (200), le dispositif distant (200) comprenant un deuxième module de transport d'image (210) pour communiquer avec le premier module de transport d'image et un module d'écriture de marque (220) pour ajouter la première marque sur l'image cible reçue.

6. Système selon la revendication 5, dans lequel le premier module de transport d'image (130) est configuré pour envoyer l'image cible prise par le module d'acquisition d'image (120) au deuxième module de transport d'image (210) qui est utilisé pour la réception de l'image cible et qui peut envoyer l'image cible avec la première marque au premier module de transport d'image (130).

7. Procédé de détermination à distance d'un point d'acuponcture, comprenant :
l'acquisition d'une image cible d'une partie du corps d'une personne cible sur laquelle le point d'acuponcture doit être déterminé ;
la transmission de l'image cible à un dispositif distant ;
la réception de l'image cible marquée d'une première marque en provenance du dispositif distant ;
la détermination de coordonnées de position du point d'acuponcture d'après l'image cible marquée de la première marque ; et
la génération d'une deuxième marque aux coordonnées de position du point d'acuponcture d'après les coordonnées de position, les coordonnées de position du point d'acuponcture étant des coordonnées tridimensionnelles, et le procédé comprenant en outre :
l'acquisition en temps réel d'une image de référence de la partie du corps de la personne cible sur laquelle le point d'acuponcture doit être déterminé ;
la décision du fait que l'image de référence est ou non cohérente avec l'image cible ; et
la conversion des coordonnées bidimensionnelles de la première marque en les coordonnées tridimensionnelles lorsque l'image de référence et l'image cible sont cohérentes.

8. Procédé selon la revendication 7, comprenant en outre :
la détermination d'une relation entre un système de coordonnées bidimensionnelles dans l'image cible et un espace tridimensionnel dans lequel se trouve la personne cible lorsque l'image de référence n'est pas cohérente avec l'image cible ; et
l'obtention des coordonnées tridimensionnelles de la deuxième marque d'après la relation.

9. Procédé selon l'une quelconque des revendications 7 à 8, comprenant en outre :
l'entraînement d'un pointeur lumineux à se déplacer pour faire en sorte que le pointeur lumineux génère la deuxième marque à la position du point d'acuponcture.

10. Procédé selon la revendication 9, dans lequel le pointeur lumineux comprend un projecteur ou un pointeur laser.
